(19) Europäisches Patentamt
European Patent Office
Office européen des brevets

(11) EP 0 556 465 B1

(12) **EUROPÄISCHE PATENTSCHRIFT**

(45) Veröffentlichungstag und Bekanntmachung des Hinweises auf die Patenterteilung:
**04.03.1998 Patentblatt 1998/10**

(51) Int Cl.[6]: **C12P 17/12**, C12N 1/20
// (C12P17/12, C12R1:05, 1:38), (C12N1/20, C12R1:05, 1:38)

(21) Anmeldenummer: **92120757.7**

(22) Anmeldetag: **04.12.1992**

(54) **Mikrobiologisches Verfahren zur Herstellung von aromatischen Hydroxy-Heterocyclus-Carbonsäuren**

Microbiological process for the preparation of aromatic hydroxylated heterocyclic acids

Procédé microbiologique pour la préparation d'acides hétérocycliques aromatiques hydroxylés

(84) Benannte Vertragsstaaten:
**AT BE CH DE ES FR GB IT LI NL SE**

(30) Priorität: **05.12.1991 CH 3572/91**

(43) Veröffentlichungstag der Anmeldung:
**25.08.1993 Patentblatt 1993/34**

(73) Patentinhaber: **LONZA AG**
**CH-3945 Gampel/Wallis (CH)**

(72) Erfinder:
- **Kiener, Andreas, Dr.**
  **Visp (Kanton Wallis) (CH)**
- **Rohner, Markus, Dr.**
  **Glis (Kanton Wallis) (CH)**
- **Heinzmann, Klaus**
  **Visperterminen (Kanton Wallis) (CH)**

(74) Vertreter: **Weinhold, Peter, Dr. et al**
**Kuhnen, Wacker & Partner,**
**Alois-Steinecker-Str. 22**
**85254 Freising (DE)**

(56) Entgegenhaltungen:
**EP-A- 0 152 948** **EP-A- 0 434 035**
**EP-A- 0 519 512** **EP-A- 0 558 022**

- **CHEMICAL ABSTRACTS, vol. 47, no. 6, 25. März 1953, Columbus, Ohio, US; abstract no. 2832h, D.E.HUGHES '6-Hydroxynicotinic acid as intermediate in oxidation of nicotinic acid by Pseudomonas fluorescens.' Spalte 2832 ; & BIOCHIM. ET BIOPHYS. ACTA Bd. 9 Seiten 226 - 227**
- **CHEMICAL ABSTRACTS, vol. 66, no. 21, 22. Mai 1967, Columbus, Ohio, US; abstract no. 94996s, HENRYK FOKS ET AL. '2-Pyrazinecarboxylic acid N-oxides. II. Reactions of the derivatives of 2-pyrazinecarboxylic acid N-oxide with acetic anhydride.' Seite 8898 ; & ACTA POL. PHARM. Bd. 23, Nr. 5 , 1966 , POL Seiten 411 - 416**
- **CHEMICAL ABSTRACTS, vol. 107, no. 25, 21. Dezember 1987, Columbus, Ohio, US; abstract no. 228395w, YAMAMOTO, TETSUYA ET AL. 'In vitro conversion of pyrazinamide into 5-hydroxypyrazinamide and that of pyrazinoic acid into 5-hydroxypyrazinoic acid by xanthine oxidase from human liver.' Seite 15 ; & BIOCHEM. PHARMACOL. Bd. 36, Nr. 19 , 1987 Seiten 3317 - 3318**
- **CHEMICAL ABSTRACTS, vol. 61, no. 2, 20. Juli 1964, Columbus, Ohio, US; abstract no. 2208f, J.C. ENSIGN ET AL. 'The pathway of nicotinic acid oxidation by a Bacillus species.' Spalte 2208 ; & J. BIOL. CHEM. Bd. 239, Nr. 7 Seiten 2285 - 2291**
- **DATABASE WPI Section Ch, Week 9250, Derwent Publications Ltd., London, GB; Class B02, AN 92-409786 & JP-A-4 304 893 (IKEDA SHOKUKEN) 28. Oktober 1992**
- **CHEMICAL ABSTRACTS, vol. 120, no. 13, 28. März 1994, Columbus, Ohio, US; abstract no. 161783u, Seite 960 ; & JP-A-5 276 967 (MITSUBISHI CHEM IND) 26. Oktober 1993**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 556 465 B1

## Beschreibung

Die Erfindung betrifft ein neues mikrobiologisches Verfahren zur Herstellung von Hydroxy-Heterocyclus-Carbonsäuren der allgemeinen Formel

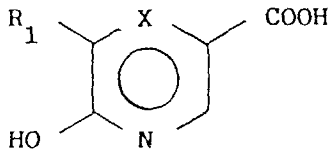

I

worin $R_1$ ein Wasserstoff- oder ein Halogenatom bedeutet und X ein Stickstoffatom oder eine $CR_2$-Funktion bedeutet, worin $R_2$ ein Wasserstoff- oder Halogenatom bedeutet, mittels Nikotinsäure oder deren lösliche Salze verwertender aerober Biomasse, ausgehend von der entsprechenden Heterocyclus-Carbonsäure oder deren löslichen Salzen.

Im folgenden werden unter Nikotinsäure auch deren lösliche Salze, insbesondere deren wasserlösliche Salze, wie beispielsweise Natriumnikotinat als Alkalisalz der Nikotinsäure, verstanden.

Diese Hydroxy-Heterocyclus-Carbonsäuren sind wichtige Zwischenprodukte zur Herstellung von Pharmazeutika. Beispielsweise ist 6-Hydroxynikotinsäure ein wichtiges Zwischenprodukt zur Herstellung von 5,6-Dichlornikotinsäure (CH-PS 664 754), welches wiederum ein Ausgangsprodukt für pharmazeutische Wirkstoffe darstellt (Setcliff et al., J. of Chem. and Eng. Data, (1976), Vol. 21, Nr. 2, S. 246).

J.C. Ensign et al. [J. Biol. Chem. 239, 2285-2291 (1964)] beschreiben eine Nikotinsäure verwertende Bacillus-Spezies. Diese Spezies metabolisiert Nikotinsäure über 6-Hydroxynikotinsäure und 2,6-Dihydroxynikotinsäure.

Die JP-A-4 304 893 beschreibt ein Verfahren zur Herstellung von 6-Hydroxynikotinsäure unter Verwendung von Nikotinsäure verwertenden Mikroorganismen der Gattungen Comamonas, Serratia, Alcaligenes und Pseudomonas.

Die EP-A-434 035 beschreibt ein Verfahren zur Herstellung von 6-Hydroxynikotinsäure mit Reinkulturen von Nikotinsäure verwertenden Mikroorganismen der Gattungen Pseudomonas, Bacillus und Achromobacter.

Eine weitere bekannte Ausführungsform für ein mikrobiologisches Verfahren zur Hydroxylierung von Nikotinsäure zur 6-Hydroxynikotinsäure ist beispielsweise in der EP-A-152 948 beschrieben. Dieses Verfahren wird derart ausgeführt, dass man zunächst Mikroorganismen mit Nikotinsäure in Gegenwart von Hefeextrakt anzüchtet und dann für die eigentliche Biotransformation die Konzentration an Nikotinsäure als Edukt so wählt, dass der Abbau der Nikotinsäure auf der Stufe der 6-Hydroxynikotinsäure gehemmt wird.

Dieses Verfahren hat ebenfalls den Nachteil, dass die Anzucht der Nikotinsäure verwertenden Mikroorganismen in Gegenwart von Hefeextrakt erfolgt, mit welchem dann die zellfreie Fermentationslösung nach der Anzucht resp. Biotransformation verunreinigt ist, was zu einer Verunreinigung der isolierten 6-Hydroxynikotinsäure führt.

Ein weiterer Nachteil besteht darin, dass dieses Verfahren mit einheitlichen (biologisch reinen) Kulturen von Mikroorganismen durchgeführt wird, die insbesondere bei Fermentationen im Grossmassstab anfälliger für Infektionen sind.

Desweiteren ist aus der EP-A-519 512 ein mikrobiologisches Verfahren zur Herstellung von 5-Hydroxypyrazincarbonsäure ausgehend von Pyrazincarbonsäure bekannt.

Bei diesem Verfahren werden einheitliche (biologisch reine) Kulturen von Mikroorganismen vor der eigentlichen Hydroxylierung mit einem Alkalisalz der Nikotinsäure angezüchtet.

Dieses Verfahren hat demnach ebenfalls den Nachteil, dass es mit einheitlichen Kulturen von Mikroorganismen durchgeführt wird, die insbesondere bei Fermentationen im Grossmassstab anfälliger für Infektionen sind.

Die Aufgabe der vorliegenden Erfindung war, diese Nachteile zu beseitigen und ein einfaches und ökologisches mikrobiologisches Verfahren zur Herstellung von Hydroxy-Heterocyclus-Carbonsäuren gemäss Formel I zur Verfügung zu stellen.

Diese Aufgabe wurde mit dem neuen Verfahren gemäss Patentanspruch 1 gelöst.

Erfindungsgemäss führt man das Verfahren derart aus, dass man a) Nikotinsäure verwertende aerobe Biomasse mit Nikotinsäure und einer Mineralsäure in einem molaren Verhältnis von Nikotinsäure zur Mineralsäure von 1 bis 8 anzüchtet, wobei über die gesamte Anzuchtphase dieses Verhältnis gewährleistet ist, und dann b) die Hydroxylierung der entsprechenden Heterocyclus-Carbonsäure der allgemeinen Formel

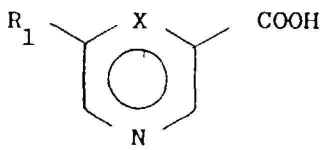

II

worin $R_1$ und X die genannte Bedeutung haben, oder deren löslicher Salze, zum gewünschten hydroxylierten Endprodukt gemäss Formel I, mit dieser Biomasse durchführt.

Unter dem Begriff "Nikotinsäure oder deren Salze verwertende aerobe Biomasse anzüchten" ist folgendes zu verstehen: Züchtet man beispielsweise aus Klärschlamm als Inokulum mit dem beschriebenen molaren Nikotinsäure-Mineralsäure-Verhältnis unter aeroben Bedingungen Biomasse an, erhält man eine Nikotinsäure verwertende aerobe Biomasse, d.h. eine Biomasse, die mit Nikotinsäure als einziger Kohlenstoff-, Stickstoff- und Energiequelle in Gegenwart von Sauerstoff wächst.

Als Inokulum können auch Erdproben aus verschiedenen Ländern angewendet werden, wie beispielsweise Erde aus dem Stadtpark in Santander (Spanien) oder Erde vom Weinberg in Visperterminen bei Visp (Schweiz).

Im Unterschied zu den bereits beschriebenen Verfahren wird das erfindungsgemässe Verfahren nicht mit einheitlichen (biologisch reinen) Kulturen von Mikroorganismen durchgeführt, sondern es wird mit Biomasse bestehend aus Mischkulturen durchgeführt.

Das molare Verhältnis von Nikotinsäure zur Mineralsäure, d.h. die Zugabe des Gemisches bestehend aus Nikotinsäure und Mineralsäure zur Zellsuspension, erfolgt derart, dass über die gesamte Anzuchtphase ein molares Verhältnis von Nikotinsäure zur Mineralsäure von 1 bis 8 gewährleistet ist.

Als Mineralsäuren können beispielsweise Schwefelsäure, Salzsäure, Salpetersäure oder Phosphorsäure angewendet werden, vorzugsweise wird Schwefelsäure angewendet.

Zweckmässig erfolgt die Zugabe des Gemisches während der Anzucht der Biomasse (Schritt a) derart, dass ein molares Verhältnis von Nikotinsäure zur Schwefelsäure von 3 bis 5 gewährleistet ist. D.h. pro mol Schwefelsäure werden zweckmässig 3 bis 5 mol Nikotinsäure zur Anzucht angewendet. Vorzugsweise werden zur Anzucht 4 bis 5 mol Nikotinsäure pro mol Schwefelsäure angewendet.

Üblicherweise erfolgt die Anzucht der Nikotinsäure verwertenden aeroben Biomasse in einem Mineralsalzmedium, vorzugsweise in dem Mineralsalzmedium, dessen Zusammensetzung in Tabelle 1 beschrieben ist.

Die Anzucht der Biomasse erfolgt zweckmässig bei einem pH-Wert von 5 bis 9, vorzugsweise von pH 6 bis 8.

Zweckmässig liegt die Temperatur während der Anzucht der Biomasse zwischen 15 und 50°C, vorzugsweise zwischen 25 und 40°C.

Üblicherweise erfolgt die Anzucht der Biomasse in einem Zeitraum von 0,5 bis 3 Tagen.

Zweckmässig werden unter diesen Bedingungen in der Anzuchtphase Mikroorganismen der Spezies Pseudomonas acidovorans DSM 7205, oder der Spezies Pseudomonas acidovorans DSM 7203, oder der Spezies Alcaligenes faecalis DSM 7204 oder Mikroorganismen mit der Bezeichnung DSM 7202 oder Mischungen von diesen angereichert, die befähigt sind, mit Nikotinsäure oder deren löslichen Salzen und einer Mineralsäure in einem molaren Verhältnis von Nikotinsäure oder deren löslichen Salzen zur Mineralsäure von 1 bis 8 zu wachsen.

Die Mikroorganismen DSM 7205, DSM 7203, DSM 7204 und DSM 7202 wurden am 13. August 1992 bei der Deutschen Sammlung von Mikroorganismen und Zellkulturen GmbH, Mascheroderweg 1b, D-3300 Braunschweig, gemäss Budapester Vertrag hinterlegt.

Diese Mikroorganismen sind aus der Literatur noch nicht bekannt und demnach ebenso Bestandteile der Erfindung. Der Mikroorganismus mit der Bezeichnung DSM 7202 konnte taxonomisch noch nicht identifiziert und einer Gattung zugeordnet werden.

Die Taxonomie der Mikroorganismen Pseudomonas acidovorans DSM 7205, DSM 7203 und Alcaligenes faecalis DSM 7204 ist nachfolgend beschrieben.

| Taxonomische Beschreibung von Pseudomonas acidovorans DSM 7205 | |
|---|---|
| Eigenschaften des Stammes | |
| Zellform | Stäbchen |
| Breite µm | 0.8-0.9 |
| Länge µm | 1.5-9.0 |
| Beweglichkeit | + |

(fortgesetzt)

| Taxonomische Beschreibung von Pseudomonas acidovorans DSM 7205 | |
|---|---|
| Eigenschaften des Stammes | |
| Geisseln | polar 1 |
| Gram-Reaktion | - |
| Lyse durch 3% KOH | + |
| Aminopeptidase (Cerny) | + |
| Sporen | - |
| Oxidase | + |
| Catalase | + |
| Wachstum | |
| anaerob | - |
| 37/41 °C | +/- |
| pH 5.7 | - |
| Mac-Conkey-Agar | + |
| SS-Agar | + |
| Cetrimid-Agar | + |
| Testosteron | - |
| Pigmente | |
| nicht diffundierend | - |
| diffundierend | - |
| fluoreszierend | - |
| Pyocyanin | - |
| Säure aus (OF-Test) | |
| Glucose aerob | ? |
| Glucose anaerob | - |
| Glucose alkalisch | + |
| Gas aus Glucose | - |
| Säure aus | |
| Glucose | - |
| Fructose | + |
| Xylose | - |
| Adonit | - |
| L-Arabinose | - |
| Cellobiose | - |
| Dulcit | - |
| Glycerol | + |
| m-Inosit | - |
| Lactose | - |

(fortgesetzt)

| Taxonomische Beschreibung von Pseudomonas acidovorans DSM 7205 | |
|---|---|
| Säure aus | |
| Maltose | - |
| Raffinose | - |
| L-Rhamnose | - |
| Salicin | - |
| D-Sorbit | - |
| Saccharose | - |
| Trehalose | - |
| Ethanol | + |
| Dulcit | - |
| ONPG/PNPG | - |
| ADH | - |
| VP | - |
| Indol | - |
| $NO_2$ aus $NO_3$ | + |
| Phenylalanindesaminase | w |
| Levan aus Saccharose | - |
| Lecithinase | - |
| Urase | - |
| Hydrolyse von | |
| Stärke | - |
| Gelatine | - |
| Casein | w |
| DNA | - |
| Tween 80 | + |
| Äsculin | - |
| PHB | - |
| Tyrosinabbau | + |
| Substratverwertung | |
| Acetat | + |
| Adipat | + |
| Caprat | + |
| Citrat | + |
| Glycolat | + |
| Lävulinat | + |
| Malat | + |

(fortgesetzt)

| Taxonomische Beschreibung von Pseudomonas acidovorans DSM 7205 | |
|---|---|
| Substratverwertung | |
| Malonat | + |
| Phenylacetat | + |
| L-Arabinose | - |
| Fructose | + |
| Glucose | - |
| Mannose | - |
| Maltose | - |
| D-Xylose | - |
| Mannitol | + |
| Gluconat | + |
| 2-Ketogluconat | + |
| N-Acetylglucosamin | - |
| L-Serin | - |
| Quinat | + |
| D,L-Tryptophan | + |
| L-Tartrat | + |
| Acetamid | + |
| α-Aminobutyrat | + |
| Ethanol | w |

| Taxonomische Beschreibung von Pseudomonas acidovorans DSM 7203 | |
|---|---|
| Eigenschaften des Stammes Zellform | Stäbchen |
| Breite µm | 0.8-1.0 |
| Länge µm | 2.6-6.0 |
| Beweglichkeit | + |
| Geisseln | polar 1 |
| Gram-Reaktion | - |
| Lyse durch 3% KOH | + |
| Aminopeptidase (Cerny) | + |
| Sporen | - |
| Oxidase | + |
| Catalase | + |
| Wachstum | |
| anaerob | - |
| 37/41 °C | +/- |
| pH 5.7 | - |
| Mac-Conkey-Agar | + |
| SS-Agar | + |
| Cetrimid-Agar | + |
| Testosteron | - |

(fortgesetzt)

| Taxonomische Beschreibung von Pseudomonas acidovorans DSM 7203 | |
|---|---|
| Wachstum | |
| | |
| Pigmente | |
| nicht diffundierend | - |
| diffundierend | - |
| fluoreszierend | - |
| Pyocyanin | - |
| Säure aus (OF-Test) | |
| Glucose aerob | ? |
| Glucose anaerob | - |
| Glucose alkalisch | + |
| Gas aus Glucose | - |
| Säure aus | |
| Glucose | - |
| Fructose | + |
| Xylose | - |
| Adonit | - |
| L-Arabinose | - |
| Cellobiose | - |
| Dulcit | - |
| Glycerol | + |
| m-Inosit | + |
| Lactose | - |
| Maltose | - |
| Raffinose | - |
| L-Rhamnose | - |
| Salicin | - |
| D-Sorbit | - |
| Saccharose | - |
| Trehalose | - |
| Ethanol | -? |
| Dulcit | - |
| ONPG/PNPG | - |
| ADH | - |
| VP | - |
| Indol | - |
| $NO_2$ aus $NO_3$ | + |
| Phenylalanindesaminase | w |

(fortgesetzt)

| Taxonomische Beschreibung von Pseudomonas acidovorans DSM 7203 | |
|---|---|
| Säure aus | |
| Levan aus Saccharose | - |
| Lecithinase | - |
| Urase | - |
| Hydrolyse von | |
| Stärke | - |
| Gelatine | - |
| Casein | + |
| DNA | - |
| Tween 80 | + |
| Äsculin | - |
| PHB | - |
| Tyrosinabbau | + |
| Substratverwertung | |
| Acetat | + |
| Adipat | + |
| Caprat | + |
| Citrat | + |
| Glycolat | + |
| Lävulinat | + |
| Malat | + |
| Malonat | + |
| Phenylacetat | + |
| L-Arabinose | - |
| Fructose | + |
| Glucose | - |
| Mannose | - |
| Maltose | - |
| D-Xylose | - |
| Mannitol | + |
| Gluconat | + |
| 2-Ketogluconat | + |
| N-Acetylglucosamin | - |
| L-Serin | - |
| Quinat | + |
| D,L-Tryptophan | + |
| L-Tartrat | + |
| Acetamid | + |
| $\alpha$-Aminobutyrat | w |
| Ethanol | + |

| Taxonomische Beschreibung von Alcaligenes faecalis DSM 7204 | |
|---|---|
| Eigenschaften des Stammes Zellform | Stäbchen |
| Breite µm | 0.6-0.8 |
| Länge µm | 1.0-2.0 |
| Beweglichkeit | + |
| Geisseln | peritrich |
| Gram-Reaktion | - |
| Lyse durch 3% KOH | + |
| Aminopeptidase (Cerny) | + |
| Oxidase | + |
| Catalase | + |
| **Wachstum** | |
| anaerob | - |
| 37/41 °C | +/+ |
| pH 5.7 | - |
| Mac-Conkey-Agar | + |
| SS-Agar | + |
| Cetrimid-Agar | + |
| **Pigmente** | |
| nicht diffundierend | - |
| diffundierend | - |
| fluoreszierend | - |
| Pyocyanin | - |
| **Säure aus (OF-Test)** | |
| Glucose aerob | ? |
| Glucose anaerob | - |
| Glucose alkalisch | + |
| Gas aus Glucose | - |
| **Säure aus** | |
| D-Glucose | - |
| D-Fructose | + |
| D-Xylose | - |
| ONPG/PNPG | - |
| ADH | - |
| VP | - |

(fortgesetzt)

| Taxonomische Beschreibung von Alcaligenes faecalis DSM 7204 | |
|---|---|
| Säure aus | |
| Indol | - |
| $NO_2$ aus $NO_3$ | + |
| Denitrifikation | - |
| Phenylalanindesaminase | - |
| Levan aus Saccharose | - |
| Lecithinase | - |
| Urease | - |
| Hydrolyse von | |
| Stärke | - |
| Gelatine | - |
| Casein | - |
| DNA | - |
| Tween 80 | - |
| Äsculin | - |
| Tyrosinabbau | + |
| Substratverwertung | |
| Acetat | + |
| Adipat | - |
| Caprat | + |
| Citrat | + |
| Glycolat | + |
| Lävulinat | - |
| D-Malat | + |
| Malonat | + |
| Phenylacetat | + |
| L-Arabinose | - |
| D-Fructose | - |
| D-Glucose | - |
| D-Mannose | - |
| Maltose | - |
| D-Xylose | - |
| Mannit | - |
| Gluconat | - |
| 2-Ketogluconat | + |
| N-Acetylglucosamin | - |
| L-Serin | - |

Im folgenden wird unter der zu hydroxylierenden Heterocyclus-Carbonsäure (Substrat) auch deren Salze, wie beispielsweise deren wasserlösliche Alkalisalze, verstanden.

Nach der Anzucht kann dann die Biomasse für die eigentliche Biotransformation (Hydroxylierung) entweder auf fachmännisch übliche Weise abgetrennt werden oder die zu hydroxylierende Heterocyclus-Carbonsäure (allgemeine Formel II) wird direkt zur angezüchteten Biomasse zugegeben.

Die eigentliche Hydroxylierung der Heterocyclus-Carbonsäure (Substrat), erfolgt auf fachmännisch übliche Weise mit nichtwachsenden Zellen.

Vorzugsweise erfolgt die eigentliche Hydroxylierung der Heterocyclus-Carbonsäure mit den in der Anzuchtphase angereicherten Mikroorganismen der Spezies Pseudomonas acidovorans DSM 7205, der Spezies Pseudomonas acidovorans DSM 7203, der Spezies Alcaligenes faecalis DSM 7204 oder mit Mikroorganismen mit der Bezeichnung DSM 7202 oder mit Mischungen von diesen.

Als Substrat können beispielsweise Nikotinsäure, Pyrazincarbonsäure oder deren halogenierte Derivate angewendet werden. Als halogenierte Derivate der Nikotinsäure oder Pyrazincarbonsäure können beispielsweise 5-Chlornikotinsäure, 4-Chlornikotinsäure oder 6-Chlorpyrazincarbonsäure angewendet werden. Vorzugsweise wird Nikotinsäure zu 6-Hydroxynikotinsäure oder Pyrazincarbonsäure zu 5-Hydroxypyrazincarbonsäure hydroxyliert .

Das Substrat kann für die Biotransformation kontinuierlich oder diskontinuierlich zugegeben werden. Zweckmässig erfolgt die Substratzugabe so, dass die Substratmenge im Fermenter 20 Gew.%, vorzugsweise 15 Gew.%, nicht übersteigt.

Als Medium können für die Hydroxylierung die fachmännisch üblichen angewendet werden, vorzugsweise entweder das in Tabelle 1 beschriebene Mineralsalzmedium oder das in Tabelle 4 beschriebene A-N-Medium.

Üblicherweise wird die Biotransformation mit Zellen durchgeführt, die eine optische Dichte bei 550 nm ($OD_{550}$) oder bei 650 nm (OD650) von 5 bis 100 aufweisen.

Zweckmässig wird die Biotransformation bei einem pH von 5 bis 9, vorzugsweise von 6,5 bis 7,5 und bei einer zweckmässigen Temperatur von 15 bis 50°C, vorzugsweise von 25 bis 35°C durchgeführt.

Nach einer üblichen Umsetzungsdauer von 5 bis 24 h kann die hydroxylierte Heterocyclus-Carbonsäure, gemäss der allgemeinen Formel I, nach fachmännisch üblichen Methoden z.B. durch Ansäuern der zellfreien Fermentationslösung oder durch Ausfällen in Form von schwerlöslichen Salzen isoliert werden. Vorzugsweise wird als hydroxylierte Heterocyclus-Carbonsäure 6-Hydroxynikotinsäure oder 5-Hydroxypyrazincarbonsäure isoliert.

Beispiel 1

(a) Anzucht der Biomasse

Die Fermentation wurde in in einem unsterilen Mineralsalzmedium (Tabelle 1) mit 1 g Nikotinsäure pro Liter, in einem Fermenter mit einem Arbeitsvolumen von 15 l bei pH 7,0, bei einer Temperatur von 30°C und einer Belüftungsrate zwischen 5 - 20 l/min durchgeführt. Zur pH-Regulierung wurde nur Säure in Form einer wässerigen Suspension bestehend aus 307 g Nikotinsäure (2,5 mol) und 49 g (0,5 mol) $H_2SO_4$ und 1 l Wasser aus einem Gefäss mit Rührer, welches am Deckel des Fermenters befestigt war, über ein pneumatisch gesteuertes Kugelventil zum Medium zudosiert. Der Fermenter wurde mit 500 ml Klärschlamm aus der Abwasserreinigungsanlage, Visp, Schweiz (Tabelle 2) beimpft. Nach 36 h wurde der Fermenter bis auf einen Liter geleert und mit frischem Medium gefüllt. Nach weiteren 24 h, 48 h und 72 h wurde dieser Vorgang wiederholt.

(b) Hydroxylierung (Herstellung von 6-Hydroxynikotinsäure)

Als die optische Dichte bei 550 nm einen Wert zwischen 5-20 erreicht hatte, wurde die Biomasse eingesetzt, um spektrophotometrisch die spezifische 6-Hydroxynikotinsäure Bildungsrate zu messen. Dazu wurde die Biomasse zuerst einmal mit 0,9% (w/v) NaCl-Lösung gewaschen. Darauf wurden 10 µl dieser Zellsuspension zu einer auf 30°C vorgewärmten Quarzküvette (1 cm Lichtweg) gegeben, die 2990 µl einer Lösung bestehend aus 6,5 g Nikotinsäure/l und 10,1 g $K_2HPO_4$/l und 4,0 g $KH_2PO_4$/l, pH 7,0, enthielt. Danach wurde die Absorption der Küvette bei 550 nm gemessen und anschliessend wurde von der gleichen Küvette die lineare Zunahme der Absorption bei 295 nm pro Minute berechnet.

Die spez. Aktivität (U) wurde nach folgender Formel bestimmt:

$$U = \frac{A_{295nm} \cdot 60}{OD_{550nm} \cdot min}$$

Die Fermentationen wurden mit einer Schlammprobe aus der Kläranlage Zermatt, mit Bodenproben aus Visperterminen und Bodenproben vom Lac de Joux, Schweiz und einer Erdprobe aus Santander, Spanien (Tabelle 2) wiederholt.

Beispiel 2

Herstellung von 5-Hydroxypyrazincarbonsäure

Die Biomasse aus Fermentationen 4,5 und 6 (Tabelle 2) wurden abzentrifugiert und einmal in 0,9% (w/v) NaCl-Lösung gewaschen. Anschliessend wurden die Zellen in einem Liter einer Lösung, enthaltend 0,5 mol (70 g) Pyrazincarbonsäure-Ammoniumsalz , pH 7,0, resuspendiert. Die optische Dichte bei 650 nm betrug dann 20. Nach einer Inkubationszeit von 16 h unter aeroben Bedingungen bei pH 7,0 und einer Temperatur von 30°C konnte mittels UV-Spektroskopie ein quantitativer Umsatz von Pyrazincarbonsäure zu 5-Hydroxypyrazincarbonsäure festgestellt werden. Die gebildete 5-Hydroxypyrazincarbonsäure wurde von den Mikroorganismen nicht abgebaut.

Als Kontrollversuch wurde Pseudomonas acidovorans D3 (DSM 4746), der sich für die industrielle Produktion von 5-Hydroxypyrazincarbonsäure besonders eignet, wie oben beschrieben angezogen und nach dem in der Europäischen Patentanmeldung 92110425.3 beschriebenen Verfahren für die Umsetzung eingesetzt. Die Resultate sind in der Tabelle 2 zusammengefasst.

Tabelle 1:

| Zusammensetzung des Mineralsalzmediums | |
|---|---|
| - $MgCl_2 \cdot 6H_2O$ | 0,8 g/l |
| - $CaCl_2$ | 0,16 g/l |
| - $Na_2SO_4$ | 0,25 g/l |
| - $K_3PO_4 . 2H_2O$ | 0,7 g/l |
| - $Na_3PO_4 \cdot 12H_2O$ | 2,4 g/l |
| - SLF | 1,0 g/l |
| - FeEDTA | 15,0 g/l |
| **Zusammensetzung der Spurenelemente (SLF) im Mineralsalzmedium** | |
| - KOH | 15,0 g/l |
| - $EDTANa_2 \cdot 2H_2O$ | 100,0 g/l |
| - $ZnSO_4 \cdot 7H_2O$ | 9,0 g/l |
| - $MnCl_2 \cdot 4H_2O$ | 4,0 g/l |
| - $H_3BO_3$ | 2,7 g/l |
| - $CoCl_2 \cdot 6H_2O$ | 1,8 g/l |
| - $CuCl_2 \cdot 2H_2O$ | 1,5 g/l |
| - $NiCl_2 \cdot 6H_2O$ | 0,18 g/l |
| - $Na_2MoO_4 \cdot 2H_2O$ | 0,2 g/l |
| **Zusammensetzung von FeEDTA:** | |
| - EDTA $Na_2 \cdot 2H_2O$ | 5,0 g/l |
| - $FeSO_4 \cdot 7H_2O$ | 2,0 g/l |
| (Der pH der Lösung wurde auf 7,0 eingestellt) | |

Tabelle 2:

| Inokulum | $OD_{550}$ vor Aktivitätsmessung | Spez. Aktivität ($A_{295} \cdot OD_{550}^{-1}$) |
|---|---|---|
| (1) ARA LONZA | 5,3 | 35,5 |
| (2) Lac de Joux | 14 | 20 |
| (3) ARA Zermatt | 9,6 | 26 |
| (4) Weinberg V'terminen | 12 | 44 |
| (5) Erde Spanien | 10 | 32 |
| (6) Kontrolle: Pseudomonas acidovorans | 3,5<br>8 | 35,1 } Doppelbestimmung<br>39 } |

Fundorte:

(1) Klärschlamm aus der Kläranlage der Fa. LONZA in Visp/Schweiz
(2) Erde vom Ufer des Lac de Joux, Le Sentier/Schweiz
(3) Klärschlamm aus der Kläranlage in Zermatt/Schweiz
(4) Erde vom Weinberg in Visperterminen bei Visp/Schweiz
(5) Erde vom Stadtpark in Santander, in Spanien

Beispiele 3-6

Aus der angezüchteten Biomasse gemäss Beispiel 1 a) konnten folgende Mikroorganismen angereichert werden:

- Pseudomonas acidovorans DSM 7205
- Pseudomonas acidovorans DSM 7203
- Alcaligenes faecalis DSM 7204
- Mikroorganismen mit der Bezeichnung DSM 7202

Diese Mikroorganismen wurden unter den nachfolgenden Bedingungen angezüchtet und für die Hydroxylierung von Nikotinsäure aus 6-Hydroxynikotinsäure eingesetzt.

Die Ergebnisse sind in Tabelle 3 zusammengefasst.

Hierzu wurden die Mikroorganismen in einem 7 l Fermenter enthaltend 5 l A-N-Medium (Tabelle 4) mit 2 g Natriumnikotinat pro 1 bei einer Temperatur von 30°C und einem pH von 7,0 angezüchtet. Zur pH Regelung wurden 5 N NaOH und 8,5% (v/v) $H_3PO_4$ verwendet. Nach 18 h Wachstum wurden zur Fermentationslösung zusätzlich 2 g Natriumnikotinat pro 1 zugegeben. Als sich die Zellen in der exponentiellen Wachstumsphase befanden, wurde die Fermentation unterbrochen und die Mikroorganismen durch Zentrifugation vom Medium getrennt. Anschliessend wurden die Zellen in 500 ml einer Lösung, enthaltend 0,27 mol (40 g) Natriumnikotinat, pH 7,0, resuspendiert. Die optische Dichte betrug dann 20. Die Hydroxylierung von Nikotinsäure zu 6-Hydroxynikotinsäure wurde spektrophotometrisch verfolgt (Tabelle 3).

Tabelle 3

| Beispiele | Benötigte Zeit für die Hydroxylierung von 0.27 mol Nikotinsäure in 500 ml | Isolierte Menge 6-Hydroxynikotinsäure nach Ansäuern der zellfreien Lösung | Ausbeute in % bezogen auf Nikotinsäure |
|---|---|---|---|
| Beispiel 3: DSM 7202 | 22 h | 15,7 g (0,11 mol) | 41% |
| Beispiel 4: DSM 7203 | 9 h | 30,1 g (0,22 mol) | 80% |
| Beispiel 5: DSM 7204 | 10 h | 28,1 g (0,2 mol) | 73% |
| Beispiel 6: DSM 7205 | 5 h | 32,0 g (0,23 mol) | 85% |

Tabelle 4:

| A-N-Medium | |
|---|---|
| Zusammensetzung | Konzentration (mg/l) |
| $Na_2HPO_4$ | 2000 |
| $KH_2PO_4$ | 1000 |
| NaCl | 3000 |
| $MgCl_2 \cdot 6H_2O$ | 400 |
| $CaCl_2 \cdot 2H_2O$ | 14·5 |
| $FeCl_3 \cdot 6H_2O$ | 0·8 |
| Pyridoxal-Hydrochlorid | $10 \cdot 10^{-3}$ |
| Riboflavin | $5 \cdot 10^{-3}$ |
| Nikotinsäureamid | $5 \cdot 10^{-3}$ |
| Thiamin-Hydrochlorid | $2 \cdot 10^{-3}$ |
| Biotin | $2 \cdot 10^{-3}$ |
| Panthotensäure | $5 \cdot 10^{-3}$ |
| p-Aminobenzoat | $5 \cdot 10^{-3}$ |
| Folsäure | $2 \cdot 10^{-3}$ |
| Vitamin B12 | $5 \cdot 10^{-3}$ |

Tabelle 4: (fortgesetzt)

| A-N-Medium | |
|---|---|
| Zusammensetzung | Konzentration (mg/l) |
| $ZnSO_4 \cdot 7H_2O$ | $100 \cdot 10^{-3}$ |
| $MnCl_2 \cdot 4H_2O$ | $90 \cdot 10^{-3}$ |
| $H_3BO_3$ | $300 \cdot 10^{-3}$ |
| $CoCl_2 \cdot 6H_2O$ | $200 \cdot 10^{-3}$ |
| $CuCl_2 \cdot 2H_2O$ | $10 \cdot 10^{-3}$ |
| $NiCl_2 \cdot 6H_2O$ | $20 \cdot 10^{-3}$ |
| $Na_2MoO_4 \cdot 2H_2O$ | $30 \cdot 10^{-3}$ |
| $EDTANa_2 \cdot 2H_2O$ | $5 \cdot 10^{-3}$ |
| $FeSO_4 \cdot 7H_2O$ | $2 \cdot 10^{-3}$ |
| (pH der Lösung wurde auf 7,0 eingestellt) | |

**Patentansprüche**

**1.** Mikrobiologisches Verfahren zur Herstellung von Hydroxy-Heterocyclus-Carbonsäuren der allgemeinen Formel

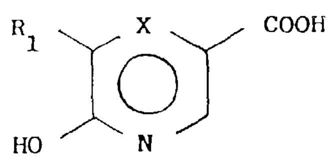

I

worin $R_1$ ein Wasserstoff- oder Halogenatom bedeutet und X ein Stickstoffatom oder eine $CR_2$-Funktion bedeutet, worin $R_2$ ein Wasserstoff- oder Halogenatom bedeutet, dadurch gekennzeichnet, dass man

(a) Nikotinsäure oder deren lösliche Salze verwertende aerobe Biomasse mit Nikotinsäure oder deren löslichen Salzen und einer Mineralsäure in einem molaren Verhältnis von Nikotinsäure oder deren löslichen Salzen zur Mineralsäure von 1 bis 8 anzüchtet, wobei über die gesamte Anzuchtphase dieses Verhältnis gewährleistet ist, und dann

(b) die Hydroxylierung der entsprechenden Heterocyclus-Carbonsäure der allgemeinen Formel

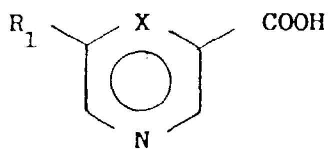

II

worin $R_1$ und X die genannte Bedeutung haben, oder deren löslichen Salze, mit dieser Biomasse durchführt.

**2.** Verfahren nach Patentanspruch 1, dadurch gekennzeichnet, dass man in Schritt a) als Mineralsäure Schwefelsäure in einem molaren Verhältnis von Nikotinsäure oder deren löslichen Salzen zur Schwefelsäure von 3 bis 5 anwendet.

**3.** Verfahren nach Patentanspruch 1 oder 2, dadurch gekennzeichnet, dass man in der Anzuchtphase in Schritt (a) Mikroorganismen der Spezies Pseudomonas acidovorans DSM 7205 und/oder der Spezies Pseudomonas acidovorans DSM 7203 und/oder der Spezies Alcaligenes faecalis DSM 7204 und/oder Mikroorganismen mit der Bezeichnung DSM 7202 anreichert und dann in Schritt (b) die Hydroxylierung mit diesen Mikroorganismen durchführt.

4. Verfahren nach einem der Patentansprüche 1 bis 3, dadurch gekennzeichnet, dass man in Schritt b) als Heterocyclus-Carbonsäure Nikotinsäure oder deren lösliche Salze zu 6-Hydroxynikotinsäure hydroxyliert.

5. Verfahren nach einem der Patentansprüche 1 bis 3, dadurch gekennzeichnet, dass man in Schritt b) als Heterocyclus-Carbonsäure Pyrazincarbonsäure oder deren lösliche Salze zu 5-Hydroxypyrazincarbonsäure hydroxyliert.

6. Verfahren nach mindestens einem der Patentansprüche 1 bis 5, dadurch gekennzeichnet, dass man die Anzucht in Schritt a) und die Hydroxylierung in Schritt b) bei einer Temperatur von 15 bis 50°C und bei einem pH von 5 bis 9 durchführt.

7. Nikotinsäure oder deren lösliche Salze verwertende Mikroorganismen der Spezies Pseudomonas acidovorans DSM 7205, der Spezies Pseudomonas acidovorans DSM 7203, der Spezies Alcaligenes faecalis DSM 7204 und Mikroorganismen mit der Bezeichnung DSM 7202, die befähigt sind, mit Nikotinsäure oder deren löslichen Salzen und einer Mineralsäure in einem molaren Verhältnis von Nikotinsäure oder deren löslichen Salzen zur Mineralsäure von 1 bis 8 zu wachsen.

## Claims

1. A microbiological process for the preparation of hydroxy-heterocyclic carboxylic acids of the general formula

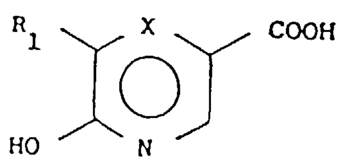

I

where $R_1$ is a hydrogen or halogen atom and X is a nitrogen atom or a group $CR_2$, where $R_2$ is a hydrogen or a halogen atom, characterised in that

(a) an aerobic biomass which utilises nicotinic acid or soluble salts thereof is cultivated with nicotinic acid or soluble salts thereof and a mineral acid in a molar ratio of nicotinic acid or its soluble salts to mineral acid of 1 to 8, whereby this ratio is maintained during the whole of the cultivating phase, and then

(b) the corresponding heterocyclic carboxylic acids of the general formula

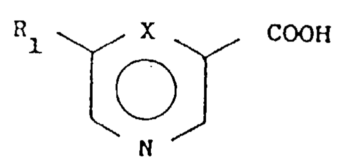

II

where $R_1$ and X have the said meanings, or their soluble salts are hydroxylated with said biomass.

2. A process according to claim 1, characterised in that, as mineral acid in step (a), sulphuric acid is used in a molar ratio of nicotinic acid or its soluble salts to sulphuric acid of 3 to 5.

3. A process according to claims 1 or 2, characterised in that microorganisms of the species *Pseudomonas acidovorans* DSM 7205 and/or of the species *Pseudomonas acidovorans* DSM 7203 and/or of the species *Alcaligenes faecalis* DSM 7204 and/or microorganisms having the designation DSM 7202 are accumulated during the cultivation phase in step (a) and then the hydroxylation is carried out in step (b) with these microorganisms.

4. A process according to one of claims 1 to 3, characterised in that, in step (b), nicotinic acid or its soluble salts, as heterocyclic carboxylic acid, is hydroxylated to 6-hydroxynicotinic acid.

5. A process according to one of claims 1 to 3, characterised in that, in step (b), pyrazinecarboxylic acid or its soluble salts, as heterocyclic carboxylic acid, is hydroxylated to 5-hydroxypyrazinecarboxylic acid.

6. A process according to at least one of claims 1 to 5, characterised in that the cultivation in step (a) and the hydroxylation in step (b) are carried out at a temperature from 15° to 50° C and at a pH of 5 to 9.

7. Microorganisms of the species *Pseudomonas acidovorans* DSM 7205, of the species *Pseudomonas acidovorans* DSM 7203 and of the species *Alcaligenes faecalis* DSM 7204 and microorganisms with the designation DSM 7202 which utilise nicotinic acid or its soluble salts, and which have the ability to grow in nicotinic acid or its soluble salts and a mineral acid in a molar ratio of nicotinic acid or its soluble salts to mineral acid of 1 to 8.

**Revendications**

1. Procédé microbiologique pour la préparation d'acides carboxyliques hétérocycliques hydroxylés de la formule générale

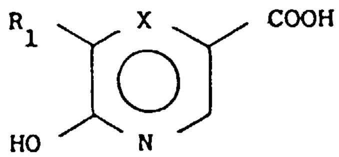

I

dans laquelle $R_1$ signifie un atome d'hydrogène ou d'halogène et X un atome d'azote ou une fonction $CR_2$, dans laquelle $R_2$ signifie un atome d'hydrogène ou d'halogène, caractérisé en ce que

(a) on met en culture l'acide nicotinique ou la biomasse aérobie utilisant ses sels solubles avec l'acide nicotinique ou ses sels solubles et un acide minéral dans un rapport molaire entre l'acide nicotinique ou ses sels solubles et l'acide minéral de 1 à 8, ce rapport étant assuré pendant la totalité de la phase de culture, et ensuite
(b) on procède à l'hydroxylation de l'acide carboxylique hétérocyclique correspondant de la formule générale

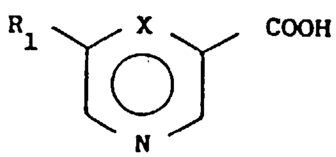

II

dans laquelle $R_1$ et X ont la signification citée ou ses sels solubles, avec cette biomasse.

2. Procédé selon la revendication 1, caractérisé en ce que l'on utilise dans l'étape (a) comme acide minéral de l'acide sulfurique dans un rapport molaire entre l'acide nicotinique ou ses sels solubles et l'acide sulfurique de 3 à 5.

3. Procédé selon la revendication 1 ou 2, caractérisé en ce que dans la phase de culture à l'étape (a), on enrichit les microorganismes de l'espèce Pseudomonas acidovorans DSM 7205 et/ou de l'espèce Pseudomonas acidovorans DSM 7203 et/ou de l'espèce Alcaligenes faecalis DSM 7204 et/ou des microorganismes portant la désignation DSM 7202 et ensuite on procède à l'étape (b) à l'hydroxylation avec ces microorganismes.

4. Procédé selon l'une des revendications 1 à 3, caractérisé en ce qu'à l'étape (b), en tant qu'acide carboxylique hétérocyclique, on hydroxyle l'acide nicotinique ou ses sels solubles en l'acide 6-hydroxynicotinique.

5. Procédé selon l'une des revendications 1 à 3, caractérisé en ce qu'à l'étape (b), en tant qu'acide carboxylique hétérocyclique, on hydroxyle l'acide carboxylique de pyrazine ou ses sels solubles en l'acide 5-hydroxypyrazincarboxylique.

6. Procédé selon au moins l'une des revendications 1 à 5, caractérisé en ce que l'on procède à la culture à l'étape

(a) et à l'hydroxylation à l'étape (b) à une température de 15 à 50°C et à un pH de 5 à 9.

7. Acide nicotinique ou microorganismes utilisant ses sels solubles de l'espèce Pseudomonas acidovorans DSM 7205, de l'espèce Pseudomonas acidovorans DSM 7203, de l'espèce Alcaligenes faecalis DSM 7204 et des microorganismes ayant la désignation DSM 7202 qui sont capables de croître avec l'acide nicotinique ou ses sels solubles et un acide minéral dans un rapport molaire d'acide nicotinique ou ses sels solubles en l'acide minéral de 1 à 8.